Europäisches Patentamt

European Patent Office

Office européen des brevets

(11) Publication number : **0 516 457 A2**

# (12) EUROPEAN PATENT APPLICATION

(21) Application number : **92304922.5**

(22) Date of filing : **29.05.92**

(51) Int. Cl.⁵ : **A61B 5/103, G01N 21/47**

(30) Priority : **29.05.91 GB 9111548**

(43) Date of publication of application :
**02.12.92 Bulletin 92/49**

(84) Designated Contracting States :
**AT BE CH DE DK ES FR GB GR IT LI NL PT SE**

(71) Applicant : **UNILEVER PLC**
**Unilever House Blackfriars**
**London EC4P 4BQ (GB)**
(84) **GB**

(71) Applicant : **UNILEVER N.V.**
**Weena 455**
**NL-3013 AL Rotterdam (NL)**
(84) **BE CH DE DK ES FR GR IT LI NL PT SE AT**

(72) Inventor : **Philp, John**
**Unilever Research Colworth Lab., Colworth House**
**Sharnbrook, Bedford MK44 1LQ (GB)**

(74) Representative : **Tonge, Robert James et al**
**UNILEVER PLC Patent Division Colworth House Sharnbrook**
**GB-Bedford MK44 1LQ (GB)**

(54) **Device for measuring the optical properties of skin.**

(57)    A device for measuring changes in pigmentation of skin which measures the reflection of light from the skin at two wavelengths, one wavelength being within the range of from 800 to 1500nm, the second wavelength being within the range of from 600 to 750nm.

EP 0 516 457 A2

Field of the Invention

The invention relates to a device for measuring in vivo properties of human skin. More particularly, the device can be used to measure changes in pigmentation of skin.

Background to the Invention and Prior Art

Changes in the pigmentation of the skin are of considerable importance in the medical and cosmetic fields as they can be predictive of pathological conditions and can be either desirable or undesirable to the consumer. It is therefore important to be able to measure such changes accurately and a variety of devices have been designed to achieve this.

Anderson R.R. and Parrish J.A. (J. Invest. Derm. (1981) 77 13-19) states that there is an optical "window" in skin between 600 and 1300nm where no absorbance is achieved and therefore the greatest penetration of skin by light occurs within this range. This is, however, only true for non-pigmented skin, Negroid Skin, in particular, will absorb within this range, especially between 600 - 700nm.

Several devices have been proposed for reflectance measurements of skin.

Of these devices, the best is the diffuse reflectance integrating sphere, as described by Diffey B.L. et al Brit. J. Derm. 1984, 111, 663-672, which allows a complete spectrum of the light reflected by the skin to be analysed. However, this instrument is costly, slow, and inconvenient to use.

Accordingly simpler instruments have been designed which measure only one, two, or sometimes three wavelengths of light. For example the chromameter (Westerhof. W. et al Photodermatol (1986) 3 310-314). However, all the light measured corresponds to the visible light part of the spectrum where pigment absorbs. Therefore none of these has been totally satisfactory because no wavelength has been used which allows the contribution of non-pigmentary colour in the skin to be measured independently of the pigmentary contribution. The results of such instruments are therefore intrinsically unreliable.

A clear need therefore exists for a simple but accurate device for measuring pigment in the skin independently of other factors affecting skin colour.

We have found, by measuring reflection of light from the skin at two wavelengths, that an accurate measurement of skin pigmentation can be achieved, provided that the first wavelength is within the near infra-red part of the spectrum ie. between 800 and 1500nm, preferably near 900nm, where no contribution to reflected light from melanin exists and the second wavelength is within the visible light part of the spectrum between 600nm and 750nm, preferably near 630nm, where pigment absorbs but blood haemoglobin does not.

Definition of the Invention

Accordingly, the invention provides a device, adapted for application to human skin, for measuring an optical property thereof, which comprises:

A device adapted for application to human skin for measuring an optical property thereof which comprises;

(i) means for illuminating the skin with light having at least two wavelengths wherein;

(a) the first wavelength falls within the range of from 800 to 1500nm; and

(b) the second wavelength falls within the range of from 600 to 750nm; and

(ii) means for measuring the light reflected from the skin originating from said two wavelengths (a) and (b).

Detailed Description of the Invention

The invention provides a device which can be applied to human skin which allows light of a defined set of wavelengths including 600-750nm and 800-1500nm and optionally other wavelength ranges to illuminate the skin and further allow the reflected light at these wavelengths to be independently measured in such a way that the ratio of the intensities of the reflected light at the two wavelengths can be calculated.

In a preferred embodiment of the invention, light emitting diodes with maximum emissions within the wavelength ranges described above, preferably wavelengths of near 900nm and near 630nm, are used to illuminate the skin alternatively. The reflected light is measured by a photodiode. The magnitude of oscillation of the measured light intensity then provides the ratio of the skin reflectively at the two wavelengths.

In a further preferred embodiment the skin is illuminated with white light, for example from a tungsten light source, and reflected light is measured by two photodiodes, one provided with a light filter allowing wavelengths between 900 and 1500nm, preferably 890-920nm, most preferably 905nm to pass, while the second is provided with a light filter allowing light between 600nm and 750nm, preferably 620-640nm, most preferably 632nm to pass. It is convenient to use glass fibre optics in either of these two embodiments to separate the light source and photodiodes from the part of the device applied to the skins.

In any embodiment of the invention it is possible to measure reflected light at further wavelengths than the two specified above in order to measure other aspects of skin colour than pigmentation, for example haemoglobin content when a wavelength of 540nm would be preferred. In all such cases the use of the 800-1500 wavelength as reference allows superior accuracy to be achieved.

In a further preferred embodiment to the invention, polarising filters are interposed between light sources and skin and between skin and light detector such that only light scattered by the skin, and hence depolarised, is measured by the detector. This eliminates error due to surface shine of the skin.

Examples

The invention is further illustrated by the following examples.

Example 1; Comparative Example A

The Effect of Erythema on the Measurement of Skin Pigment

Where skin colour measuring devices are restricted to the visible range of light only, the unequivocal assignment of a measured change to either redness (erythema) or tanning (pigmentation) is extremely difficult.

A study of the effect of induced reddening of local areas of Caucasian skin in situ was undertaken. Control skin sites were located on the outer forearm where tanning was obvious and on the inside of the upper arm which showed little evidence of tanning. An erythema was raised by slapping each moderately firmly with the fingers of one hand. In order to determine the maximum range of the output from the device used when measuring all shades of skin a white Caucasian and dark brown African were included as controls.

Example A

A Dia-stron Erythema meter optically modified to measure at wavelengths 905nm and 632nm was used to measure both redness as Erythemal index and tanning as Melanotic Index. The wavelengths were selected using narrow bandpass interference filters (approx 5 to 10nm). Results are given in table 1.

## Table 1

| Skin type | Erythemal Index | Melanotic Index |
|---|---|---|
| **Control** | | |
| White | – | –125.8 +/– 1.8 |
| Black | – | +374 +/–12.9 |
| **Experimental** | | |
| Upper arm; no erythema | 71.8 +/– 14.3 | –87.8 +/– 13.7 |
| Upper arm with erythema | 131.3 +/– 17.1 | –97.5 +/– 4.1 |
| Forearm; no erythema | 103 | –50.3 +/– 1.5 |
| Forearm with erythema | 192 +/– 13.9 | –59.7 +/– 5.5 |

Values are the mean +/- standard deviation

Comparative Example A

A commercially available colour measuring meter (Minolta Chromameter) which measures colour change as total intensity of reflected light (L), the difference between red and green (a), and the difference between yellow and blue (b). The chromameter operates with broad bandpass filters with the transmission maximum near 640nm for the red, 540nm for the green and 450nm for the blue. Results are given in table 2.

Table 2

| Skin Type | L | a | b |
|---|---|---|---|
| Control | | | |
| White | 71.0+/-0.7 | 3.5+/-1.4 | 10.5+/-0.2 |
| black | 3.9+/-0.7 | 7.89+/-0.1 | 5.9+/-1.0 |
| Experimental | | | |
| upper arm; no erythema | 69.0+/-0.5 | 4.5+/-0.4 | 9.7+/-0.2 |
| upper arm, erythema | 65.5+/-0.6 | 9.7+/-0.4 | 7.8+/-0.8 |
| forearm; no erythema | 63.5+/-0.4 | 5.7+/-0.4 | 14.6+/-1.0 |
| forearm; erythema | 60.2+/-0.6 | 10.3+/-0.5 | 12.7+/-0.4 |

Conclusions

It is obvious from the results that the new optical method using the difference between near infrared light reflection and red light reflection offers a greater dynamic range ie. white to brown skin of approximately -126 to +370 while the chromameter only gives 71 to 39.

Comparing control skin with the same area after erythema induction shows that the melanotic index values show little change. The difference between the white skin of the upper arm and the slightly tanned skin of the forearm stayed at approximately 38. This was not the case for L, a or b values obtained with the Chromameter. This is good evidence for the claim the changes in the level of other light absorbing materials in the skin do not affect the measurement of skin pigment.

**Claims**

1. A device adapted for application to human skin for measuring an optical property thereof which comprises;
   (i) means for illuminating the skin with light having at least two wavelengths; and
   (ii) means for measuring the light reflected from the skin originating from the two wavelengths;
      characterised in that
      (a) the first wavelength falls within the range of from 800 to 1500nm; and
      (b) the second wavelength falls within the range of from 600 to 750nm.

2. A device according to claim 1 wherein:
   (a) the first wavelength is 890-920nm; and
   (b) the second wavelength is 620-640nm.

3. A device according to claim 1, which is intended for measuring changes in skin pigmentation.